(19) Europäisches Patentamt

European Patent Office

Office européen des brevets

(11) **EP 0 879 621 B1**

(12) **EUROPÄISCHE PATENTSCHRIFT**

(45) Veröffentlichungstag und Bekanntmachung des Hinweises auf die Patenterteilung:
**14.01.2004 Patentblatt 2004/03**

(51) Int Cl.7: **A61N 1/368**

(21) Anmeldenummer: **98250155.3**

(22) Anmeldetag: **06.05.1998**

(54) **Vorrichtung zum Nachweis ventrikulärer Tachykardie**

Device for detection of ventricular tachycardia

Dispositif pour la détection de la tachycardie ventriculaire

(84) Benannte Vertragsstaaten:
**CH DE FR LI NL**

(30) Priorität: **07.05.1997 US 852406**

(43) Veröffentlichungstag der Anmeldung:
**25.11.1998 Patentblatt 1998/48**

(73) Patentinhaber: **BIOTRONIK Mess- und Therapiegeräte GmbH & Co Ingenieurbüro Berlin 12359 Berlin (DE)**

(72) Erfinder: **Nigam, Indra B.
Lake Oswego, Oregon 97035 (US)**

(74) Vertreter: **Eisenführ, Speiser & Partner
Patentanwälte Rechtsanwälte
Anna-Louisa-Karsch-Strasse 2
10178 Berlin (DE)**

(56) Entgegenhaltungen:
**EP-A- 0 094 758        EP-A- 0 617 980
WO-A-93/02746        US-A- 5 107 850**

**Beschreibung**

Die Erfindung betrifft eine Vorrichtung der im Oberbegriff des Anspruchs 1 angegebenen Art. Eine solche Vorrichtung ist als eigenständiges Diagnosegerät oder als Bestandteil eines Herzschrittmachers mit Antitachykardie-Betriebsart, Defibrillators oder Kardioverters realisierbar.

Die Therapie tachykarder Herzrhythmusstörungen ist eines der wichtigsten Aufgabengebiete der Kardiologie und speziell ein Hauptanwendungsfeld der Elektrostimulation des Herzens. Im Hinblick auf die vielfältigen Erscheinungsformen und Ursachen derartiger Störungen werden seit Jahren umfangreiche Anstrengungen unternommen, die Voraussetzungen für einen therapeutischen Erfolg durch feinere Klassifizierung der tachykarden Arrhythmien und exaktere Zuordnung zu typischen Defekten des kardialen Reizleitungssystems zu verbessern.

Aufgrund der weiten Verbreitung und der Behandlungsbedürftigkeit pathologischer ventrikulärer Tachykardien (nachfolgend auch als "VT" bezeichnet) einerseits und der guten Chancen für ihre erfolgreiche Behandlung mittels spezieller Schrittmacherimpulsfolgen andererseits hat sich dabei die sichere Unterscheidung von anderen Arrhythmien, speziell von physiologischen Tachykardien, ventrikulären Fibrillationen ("VF") oder supraventrikulärer Tachykardie ("SVT"), - auch unter speziellen Umständen - als besonders wichtiges Problem herauskristallisiert.

In DE-A-44 39 256 wird vorgeschlagen, die relative Verteilung von als Fibrillations-Intervalle bzw. als Tachykardie-Intervalle anzusehenden Herzschlagintervallen innerhalb vorbestimmter Zeitbereiche zur Klassifizierung des vorherrschenden Arrhythmientyps zu nutzen. Damit soll der Tatsache Rechnung getragen werden, daß die Intervallängen sich bei ventrikulärer Tachykardie einerseits und Fibrillationen andererseits in der Praxis häufig erheblich überlappen.

Eine ganze Reihe anderer Lösungsansätze - die hier nicht im einzelnen erörtert werden sollen - will sich der Analyse der räumlichen Ausbreitung bzw. Korrelation von Depolarisationen im Herzgewebe bedienen. Dies erfordert die Implantation einer Vielzahl von Elektroden zur Signalerfassung - zum Teil sogar perikardial - und hat schon aus diesem Grunde nur geringe Realisierungschancen.

In US-A-4 860 749 wird ein Verfahren zur Unterscheidung einer ventrikulären von einer Sinus- bzw. anderen supraventrikulären Tachykardie beschrieben, bei dem die atriale und die ventrikuläre Herzrate (deren jeweilige Kehrwerte werden im weiteren auch als "PP-Intervall" bzw. "RR-Intervall" bezeichnet) sowie das AV-Intervall (im weiteren teilweise auch als "PR-Intervall" bezeichnet) gemessen werden. Liegt das RR-Intervall innerhalb eines vorbestimmten Bereiches und ist es kürzer als das PP-Intervall, wird der Zustand ohne weiteres als ventrikuläre Tachykardie klassifiziert. Sind die atriale und die ventrikuläre Rate infolge 1:1-AV-Überleitung oder retrograder Überleitung etwa gleich, wird das gemessene AV-Intervall einem Vergleich mit einem vorgegebenen Wert ("Sinus-AV-Intervall") unterzogen und aus dem Ergebnis des Vergleichs das Klassifizierungskriterium gewonnen.

In US-A-5 325 856 wird ein Verfahren zur Unterscheidung zwischen ventrikulären und supraventrikulären Tachykardien vorgeschlagen, das auf einem Vergleich der zeitlichen Divergenz der PR- und der RR-Werte mit zwei vorbestimmten Schwellwerten beim Einsetzen der Tachykardie beruht.

US-A-5 327 900 beschreibt ein Verfahren zur Unterscheidung zwischen pathologischen und physiologischen (Sinus-) Tachykardien bei vergleichbarer atrialer und ventrikulärer Rate, das auf der Zuordnung des gemessenen AV-Intervalls zu einem vorgegebenen AV-Zeitfenster beruht, welches anhand des AV-Intervalls bei normalem Sinusrhythmus bestimmt worden ist. Dieser Algorithmus ist vergleichsweise einfach; es liegen jedoch bislang keine Belege für eine hinreichende Unterscheidungs-Effizienz vor.

In EP-A-0 597 459 wird ein Verfahren beschrieben, bei dem im Falle einer Übereinstimmung von RR- und PP-Intervall zunächst ein Vergleich der Länge des AV- (PR-) Intervalls mit einem vorgegebenen Basiswert und schließlich - falls dies nicht zu einem schlüssigen Ergebnis führt - eine Test-Stimulation im Atrium ausgeführt und die Klassifizierung anhand der stimulierten Herzantwort, insbesondere zeitlicher Änderungen der RR-Intervalle, vorgenommen wird.

Eine Vorrichtung zum Nachweis ventrikulärer Tachykardie unter Berücksichtigung der zeitlichen Änderung zwischen aufeinander folgenden PP-Intervallen ist aus WO 93/02746 bekannt.

Der Erfindung liegt die Aufgabe zugrunde, eine Vorrichtung anzugeben, die eine zuverlässige Erkennung einer - einen adäquaten Therapie-Eingriff erfordernden - ventrikulären Tachykardie mit vertretbarem technischem Aufwand und unter geringer Belastung für den Patienten ermöglicht. Die Vorrichtung soll speziell eine verbesserte Erkennung einer ventrikulären Tachykardie bei gleichzeitigem Vorliegen einer atrialen Tachyarrhythmie ermöglichen.

Diese Aufgabe wird durch eine Vorrichtung mit den Merkmalen des Anspruchs 1 gelöst.

Die Erfindung schließt den Gedanken ein, eine Vorrichtung anzugeben, die Mittel umfaßt, um allein aufgrund der mit je einem Fühler im Atrium und im Ventrikel aufgenommenen Signale spontaner Herzaktivität einen mehrstufigen Klassifizierungsvorgang auszuführen, bei dem die Länge der RR-, PP- und erforderlichenfalls auch der PR-Intervalle sowohl betragsmäßig als auch in der zeitlichen Entwicklung (hinsichtlich ihrer Stabilität, Regularität oder der Monotonie einer zeitlichen Änderung) grundsätzlich auf einer Schlag-zu-Schlag-Basis ausgewertet wird.

Da hierbei weder eine Bestimmung und Analyse der EKG-Kurvenform noch der räumlichen Ausbreitung der Depolarisationen im Herzgewebe erforderlich ist, ist diese Vorrichtung in Herstellung und Implantation vergleichsweise unaufwendig und bringt bei der Implantation und im Betrieb für den Patienten keine höhere. Belastung mit sich als ein herkömmlicher Herzschrittmacher/Kardioverter.

Für jedes abgelaufene RR-Intervall wird zunächst durch eine Schwellwertdiskrimination festgestellt, ob seine Länge die Einordnung in den Ratenbereich einer ventrikulären Tachykardie ("VT-Zone") rechtfertigt. Ist dies der Fall, so wird eine Reihe weiterer Kriterien angewandt. Es handelt sich speziell um die Kriterien

  (a) plötzliches Einsetzen,
  (b) Intervallstabilität und PR-Intervall-Regularität,
  (c) Monotonie der zeitlichen Änderung der PR-Intervalle,
  (d) Verhältnis zwischen RR- und PP-Intervalllänge,

von denen im Normalfall mehrere, aber nicht unbedingt alle geprüft werden müssen. Zum Teil erfolgt die Prüfung zweckmäßigerweise an Intervall-Mittelwertn, die unter Einbeziehung mindestens dreier vorangegangener Intervalle gebildet wurden. Wie diese Kriterien definiert sind und geprüft werden, wird weiter unten erläutert.

So liegt eine VT vor, wenn (bei in die VT-Zone fallender RR-Intervalllänge) der Mittelwert der RR-Intervalllänge kleiner als derjenige der PP-Intervalllänge ist.

Ist hingegen die PP-Intervalllänge kleiner als die RR-Intervalllänge, so ist zusätzlich die Stabilität der RR-Intervalle zu prüfen und der Verhältniswert aus RR- und PP-Intervalllänge zu bilden. Ist Stabilität gegeben, und ist die RR-Intervalllänge nicht ein Mehrfaches der PP-Intervalllänge, so liegt hier ebenfalls eine VT vor.

Sind die RR- und PP-Intervalllängen annähernd gleich, so sind zusätzlich die PR-Intervalle auszuwerten. Es liegt eine VT vor, wenn

- die RR-Intervalle nicht stabil und die PR-Intervalle nicht regulär sind oder
- die RR-Intervalle, aber nicht die PP-Intervalle stabil sind oder
- sowohl die RR- als auch die PP-Intervalle stabil sind und sich die PR-Intervalle monoton ändern oder
- sowohl die RR- als auch die PP-Intervalle stabil sind und ein plötzliches Einsetzen auftrat.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

  Figur 1 ein Funktions-Blockschaltbild einer Vorrichtung nach einer Ausführungsform der Erfindung,

  die Figuren 1a und 1b schematische Darstellungen zum inneren Aufbau von Funktionseinheiten der Vorrichtung nach Fig. 1,

  die Figuren 2a bis 2c ein Flußdiagramm des Vorgehens zum Nachweis einer ventrikulären Tachykardie mittels einer Vorrichtung nach Fig. 1 und

  Figur 3 eine Detaildarstellung zu einer Ausführung der Ablaufsteuerung 3 aus Fig. 1.

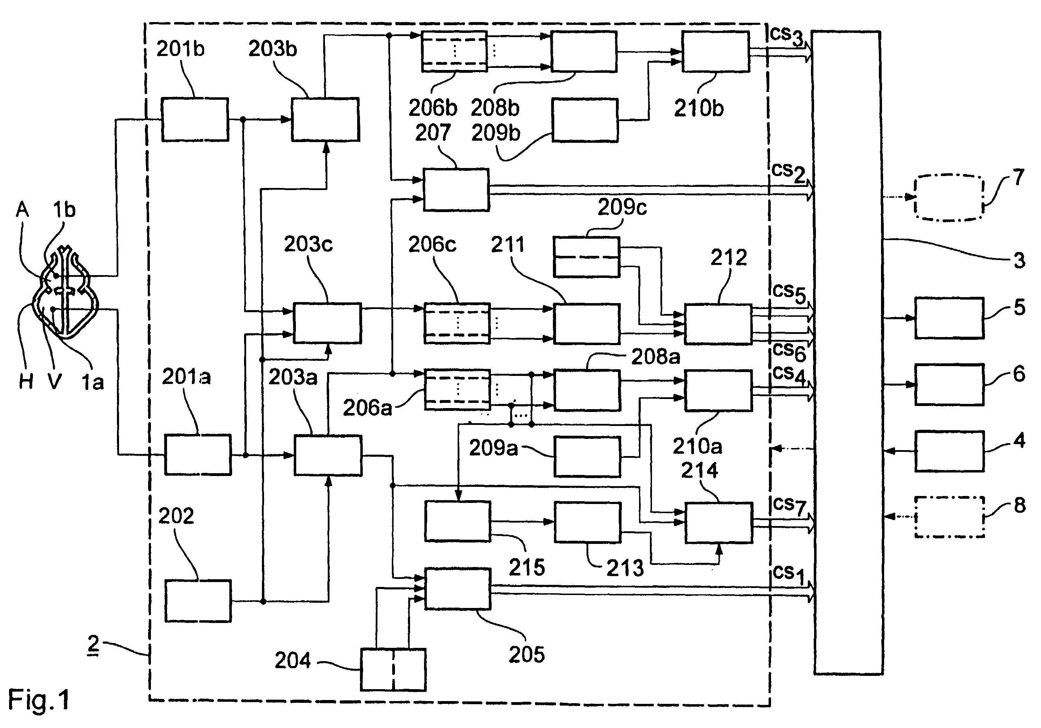

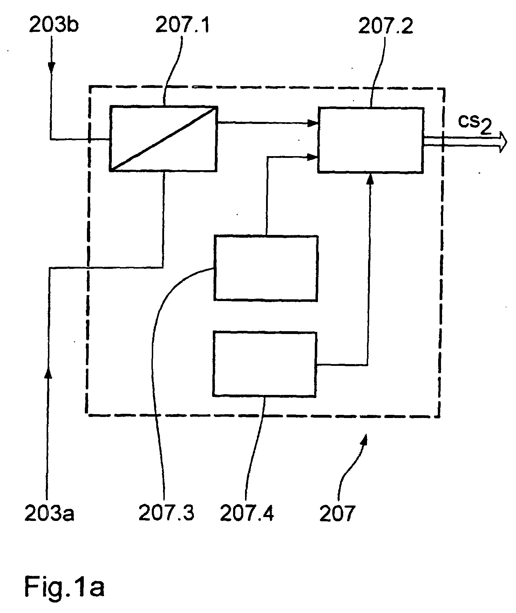

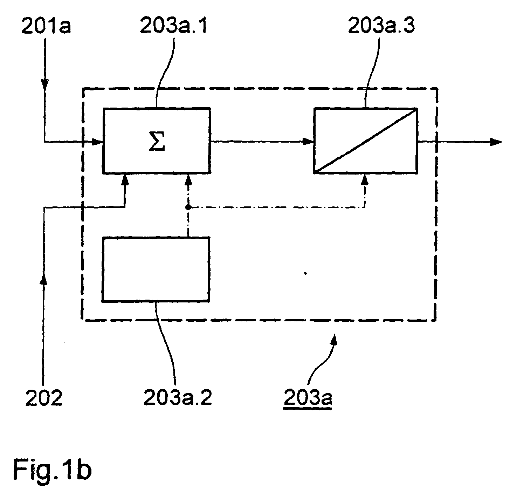

In Figur 1 ist schematisch der Aufbau einer über eine Ventrikel-Elektrode 1a und eine Atrium-Elektrode 1b als endokardiale Signalaufnehmer mit einem Herzen H verbundenen Vorrichtung 2 zur Tachykardieklassifizierung, speziell zum Nachweis ventrikulärer Tachykardie, nach einer Ausführungsform der Erfindung anhand der wesentlichen Funktionselemente dargestellt.

Die Vorrichtung 2 wird über eine Ablaufsteuerung (einen Controller) 3 entsprechend einem weiter unten erläuterten, in einem Programmspeicher 4 gespeicherten Ablauf gesteuert. Aufgrund von durch die Vorrichtung ermittelten Klassifizierungsdaten wird wahlweise ein - als solcher bekannter - Herzschrittmacher 5 oder Defibrillator 6 zur Einleitung einer geeigneten Elektrotherapie angesteuert. Die von der Vorrichtung 2 ausgegebenen Klassifizierungsdaten können auf einer optional vorgesehenen (mit gepunkteten Linie symbolisierte) Anzeigeeinheit 7 dargestellt und somit erforderlichenfalls auch unabhängig von der Einleitung einer Elektrotherapie - beispielsweise zur Festlegung einer medikamentösen Therapie - genutzt werden. Ebenfalls optional ist eine Eingabeeinheit 8 zur Eingabe von die Ausführung einer konkreten Klassifizierungsprozedur betreffenden, patientenspezifischen Daten vorgesehen.

Die Vorrichtung 2 wird bevorzugt zusammen mit dem Controller 3 als implantierbare Vorrichtung und als integraler Bestandteil eines automatischen Herzschrittmachers/Defibrillators ausgeführt und ist in diesem Fall über bekannte (nicht dargestellte) Telemetrieeinrichtungen mit der Anzeige- und der Bedieneinheit 7, 8 verbunden, die speziell in einem externen Programmiergerät enthalten sind. Dies erlaubt die ständige zeitnahe Überwachung der Herzaktivitäten eines Patienten auf das Auftreten und die Art tachykarder Rhythmusstörungen hin und die sofortige Einleitung einer adäquaten Elektrotherapie.

Zumindest Teile der Vorrichtung 2 und/oder der Controller 3 können aber für spezielle Anwendungen - etwa für den begleitenden Einsatz bei herzchirurgischen Eingriffen - auch in einem externen Gerät realisiert sein.

In beiden Fällen wird die praktische Ausführung in vorteilhafter Weise in einer Mikroprozessorstruktur oder auch partiell auf ASIC(Kundenwunschschaltungs)-Basis erfolgen, wobei die nachfolgend erläuterten Funktionselemente zumindest teilweise durch Software realisiert sind.

Wie in Fig. 1 dargestellt, weist die Vorrichtung 2 einen mit der atrialen Abfühlelektrode 1b verbundenen Abfühlverstärker 201b zum Abfühlen elektrischer Aktivität (der P-Wellen) im Vorhof A des Herzens H und einen mit der ventrikulären Abfühlelektrode 1a verbundenen Abfühlverstärker 201a zum Abfühlen elektrischer Aktivität (insbesondere der R-Wellen bzw. QRS-Komplexe) in der Herzkammer V des Herzens H auf. Weiter ist ein Zeit- bzw. Taktgeber 202 zur Erfassung des Zeitpunktes des Auftretens der jeweiligen elektrischen Aktivität vorgesehen.

Eine eingangsseitig mit der ventrikulären Elektrode 1a sowie dem Zeitgeber 202 verbundene erste Berechnungseinheit 203a ermittelt die (gemittelten) Zeitintervalle zwischen jeweils aufeinanderfolgenden elektrischen Aktivitäten in der Herzkammer (RR-Intervalle). Sie stellt in der einfachsten Ausführung im Grunde einen R-Wellen-getriggerten Zähler für die Impulse des Zeitgebers dar, führt in der bevorzugten Ausführung jedoch eine Mittelwertbildung der Zählwerte über eine Anzahl von R-Wellen aus.

Analog dient eine eingangsseitig mit der Atrium-Elektrode 1b sowie dem Zeitgeber 202 verbundene zweite Berechnungseinheit (bzw. in der einfachsten Ausführung ein P-Wellengetriggerter Zähler) 203b zur Ermittlung und zeitlichen Mittelung der PP-Intervalle. Die Mittelung der gemessenen Zeitintervalle in der ersten und zweiten Berechnungseinheit 203a. 203b erfolgt auf an sich bekannte Weise durch eine (nicht dargestellte) Integrations- bzw. Mittelungseinrichtung, wobei beispielsweise eine Anzahl von vier erfaßten Herzaktionen zugrundegelegt wird (vgl. Fig. 1b).

Ein Zwei-Bereichs-Schwellwertspeicher 204 dient zur Speicherung eines unteren und eines oberen Schwellwertes für die RR-Intervalle (bzw. RR-Intervall-Mittelwerte), von denen der obere einen Bereich normaler Herzaktivität von einem Bereich tachykarder Rhythmusstörungen abgrenzt und der untere einen Bereich eindeutig als ventrikuläre Fibrillation zu klassifizierender Herzaktivität nach oben hin abgrenzt. Mit den Ausgängen der ersten Berechnungseinheit 203a und des Schwellwertspeichers 204 ist ein Zwei-Stufen-Komparator 205a zum Vergleich der Ventrikel-Zeitintervalle (oder -Zeitintervall-Mittelwerte) mit dem unteren und oberen Schwellwert und zur Ausgabe eines das Vergleichsergebnis - d.h. die Lage der ermittelten RR-Intervalle in einem der oben erwähnten Bereiche - kennzeichnenden ersten Klassifizierungssignals $cs_1$ verbunden.

Mit den Ausgängen der ersten und zweiten Berechnungseinheit 203a, 203b sind ein erster bzw. zweiter Intervallspeicher 206a, 206b zur Speicherung einer vorbestimmten Anzahl berechneter Ventrikel- bzw. Atrium-Zeitintervalle bzw. Zeitintervall-Mittelwerte sowie eine weitere Komparatorstufe 207 zum Vergleich der Atrium-Zeitintervalle mit den Ventrikel-Zeitintervallen und zur Ausgabe eines das Vergleichsergebnis kennzeichnenden zweiten Klassifizierungssignals $cs_2$ verbunden. Die Intervallspeicher 206a, 206b sind serielle Speicher und insbesondere nach dem LI-FO-Prinzip organisiert. Der Aufbau der Komparatorstufe 207 ist in Fig. 1a genauer gezeigt und wird weiter unten beschrieben.

Weiterhin ist mit dem Ausgang des ersten Intervallspeichers 206a eine dritte Berechnungseinheit 208a und mit dem Ausgang des zweiten Intervallspeichers 206b eine vierte Berechnungseinheit 208b verbunden. Die dritte und vierte Berechnungseinheit führen eine Berechnung der zeitlichen Änderungen zwischen aufeinanderfolgenden, in den Intervallspeichern vorab abgespeicherten RR- bzw. PP-Intervallen (bzw. Intervall-Mittelwerten) aus und stellen an ihrem Ausgang die entsprechenden Differenz- bzw. Quotientenwerte bereit.

Ein erster und zweiter Kriterienspeicher 209a, 209b dienen zur Speicherung jeweils eines vorgegebenen Stabilitätskriteriums für die zeitliche Änderung zwischen aufeinanderfolgenden RR- bzw. PP-Intervallen. Eine eingangsseitig mit dem Ausgang der dritten bzw. vierten Berechnungseinheit 208a, 208b und dem jeweils zugehörigen Kriterienspeicher 209a, 209b verbundene Komparatorstufe 10a, 210b dient zum Vergleich der berechneten zeitlichen Änderung der PP- bzw. RR-Intervalle mit dem gespeicherten zugehörigen Stabilitätskriterium und zur Ausgabe eines das vergleichsergebnis kennzeichnenden dritten und vierten Klassifizierungssignals $cs_3$ bzw. $cs_4$.

Eine fünfte Berechnungseinheit 203c, die in der einfachsten Ausführung als durch aufeinanderfolgende P- und R-Wellen getriggerter Zähler aufgebaut ist, ist eingangsseitig sowohl mit der Atrium-Elektrode 1b als auch mit der Ventrikel-Elektrode 1a und weiterhin mit dem Zeitgeber 202 verbunden und bestimmt die - ggfs. zeitlich gemittelten - PR-Intervalle, vielfach auch als AV-Intervalle bezeichnet. Mit dem Ausgang der fünften Berechnungseinheit 203c ist ein dritter Intervallspeicher 206c für die PR- bzw. Av-Intervalle (oder Mittelwerte aus diesen) verbunden. Mit dem Ausgang dieses Speichers 206c ist eine sechste Berechnungseinheit 211 zu Berechnung der zeitlichen Änderung der PR-Intervalle und zur Ausgabe eines Differenzwertes oder Quotienten als Maß für die zeitliche Änderung verbunden.

Ein dritter, als Mehrbereichsspeicher organisierter, Kriterienspeicher 209c speichert zum einen ein vorgegebenes Regularitäts- und zum anderen ein vorgegebenes Monotoniekriterium für die zeitliche Änderung zwischen aufeinanderfolgenden PR-Intervallen. (Natürlich können hierfür auch getrennte Speicher vorgesehen sein.) Eine eingangsseitig mit dem Ausgang der fünften Berechnungseinheit 211 und dem dritten Kriterienspeicher 209c verbundene zweistufige Vergleicher- und Berechnungseinheit 212 führt einen Vergleich der berechneten zeitlichen Änderung der PR-Intervalle mit dem gespeicherten Regularitätskriterium bzw. -

wenn der Vergleich in der ersten Stufe zeigt, daß diese nicht erfüllt ist - mit dem weiterhin in 209c gespeicherten Monotoniekriterium aus und gibt ein das Vergleichsergebnis kennzeichnendes fünftes bzw. sechstes Klassifizierungssignal $cs_5$ bzw. $cs_6$ aus, das die Regularität resp. Monotonie des Zeitverhaltens des PR-Intervalls ausdrückt.

Der vierten Berechnungseinheit 208a ist ein weiterer Speicher 213 zur Speicherung eines Ventrikel-Vergleichsintervalles zugeordnet.

Eingangsseitig ist mit den Ausgängen der ersten Berechnungseinheit 203a einerseits und des ersten Intervallspeichers 206a andererseits sowie - über einen zusätzlichen Eingang - mit dem Vergleichswertspeicher 213 eine weitere Komparatoreinheit 214 verbunden, die einen Vergleich des aktuellen Ventrikel-Zeitintervalls (oder -Zeitintervall-Mittelwertes) mit einem gespeicherten Ventrikel-Vergleichsintervall ausführt und daraufhin ein siebentes Klassifizierungssignal $cs_7$ ausgibt. Der Vergleichswertspeicher 213 ist bei der dargestellten Ausführung eingangsseitig mit einer Vergleichswert-Berechnungseinheit 215 verbunden, die ihrerseits eingangsseitig mit dem Ausgang der ersten Speichereinheit 206a verbundenen ist und die laufend anhand der letzten in der Vergangenheit bestimmten (im Speicher 206a abgespeicherten) RR-Intervalle den für den Vergleich im Komparator 214 maßgeblichen Ventrikel-Differenzbetrag berechnet.

Fig. 1a zeigt den inneren Aufbau der Komparatorstufe 207: Mit den Ausgängen der (in dieser Figur nicht gezeigten) Berechnungseinheiten 203a und 203b sind die beiden Eingänge einer Dividierstufe 207.1 verbunden, die eine Division des jeweils zugeführten RR-Intervalles durch das zugehörige PP-Intervall ausführt und deren Ausgang mit einem Eingang einer Vergleicherstufe 207.1 verbunden ist, dem sie den errechneten aktuellen Wert dieses Quotienten zuführt. Der andere Vergleichssignaleingang der Vergleicherstufe ist mit einem Quotientenspeicher 207.3 verbunden, in dem fest vorgegebene (ganzzahlige) Werte für den RR/PP-Quotienten gespeichert sind und der daher als Festwertspeicher (ROM) ausgeführt sein kann. In einem weiteren, bevorzugt als reprogrammierbarer Speicher (etwa EEPROM) ausgebildeten, Speicher 207.4 ist ein Quotienten-Toleranzwert gespeichert, der über einen dritten Eingang an der Vergleicherstufe 207.2 bereitgestellt wird. Das an deren Ausgang abgegebene zweite Klassifizierungssignal $cs_2$ drückt - in den Grenzen des vorgegebenen Toleranzbereiches - somit aus, ob der Quotient RR-Intervall/PP-Intervall ganzzahlig ist.

Fig. 1b ist eine schematische Darstellung des Aufbaus der Berechnungseinheiten 203a und 203b in der Ausführung mit zeitlicher Mittelung der gemessenen Intervallwerte, dargestellt am Beispiel der Einheit 203a. Das vom Ventrikel-Leseverstärker 201a zugeführte Herzaktionssignal und das Zeit- bzw. Taktsignal des Zeitgebers 202 sowie ein in einem Festwertspeicher 203a.2 gespeicherter Intervallzahlwert n (z.B. n = 4) steuern einen Zähler 203a.1, wobei die Zeitgebersignale dem Zähleingang zugeführt und solange gezählt werden, bis die n-te erfaßte R-Welle als ResetSignal verarbeitet wird, womit der Zähler 203.al die Gesamtzeitdauer von n RR-Intervallen bestimmt und auf einen Eingang einer Dividierstufe 203a.3 ausgibt. Deren anderer Eingang ist mit dem Ausgang des Intervallzahlspeichers 203a.2 verbunden derart, daß sie eine Division der Gesamtzeitdauer durch die Intervallzahl durchführt und somit die über n Intervalle gemittelte RR-Intervallänge liefert. Die Berechnungseinheit 203b ist in Aufbau und Funktion analog ausgebildet, aber mit dem Atrium-Leseverstärker 201b verbunden und bestimmt die mittlere PP-Intervalldauer.

Bei der dritten Berechnungseinheit 203c erfolgt die Bestimmung der zeitlich gemittelten PR-Intervalle auf entsprechende Weise, wobei hier die erfaßten P- und R-Wellen getrennte Start- und Stop-/Reset-Signale eines Intervalllängen-Zählers bilden.

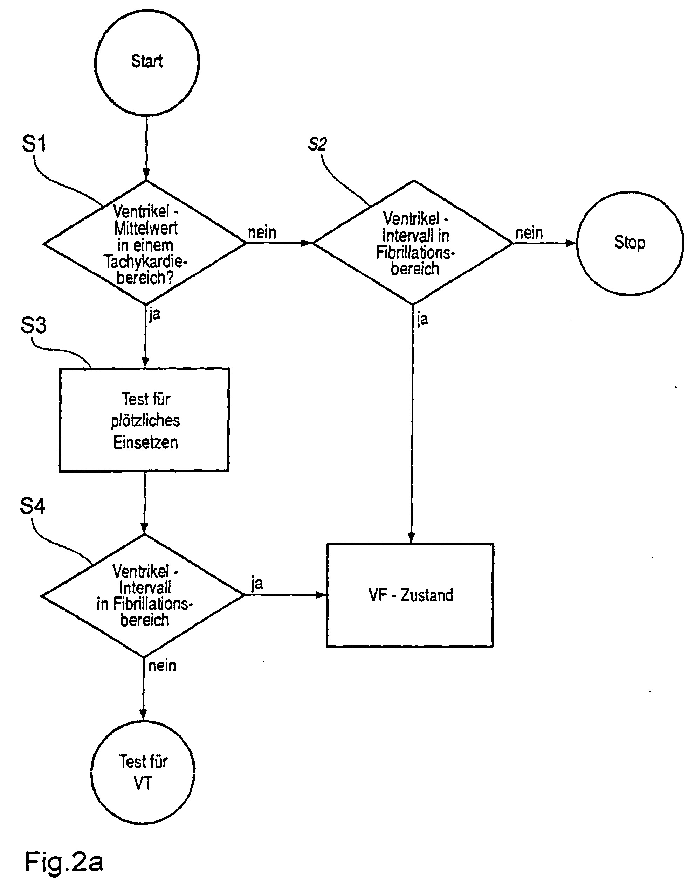

Der Betrieb der oben in ihrem grundsätzlichen Aufbau beschriebenen Vorrichung wird nachfolgend anhand der Figuren 2a bis 2c erläutert, die zusammen ein Flußdiagramm des Vorgehens zum Nachweis einer ventrikulären Tachykardie bzw. zu deren Unterscheidung von einer Sinus- oder anderen supraventrikulären Tachyarrhytbmie zeigen. Dieses Vorgehen ist besonders vorteilhaft bei gleichzeitigem Vorliegen einer atriellen Tachyarrhythmie, die mit gleicher oder höherer Rate abläuft wie die im Ventrikel nachgewiesene und zu klassifizierende Tachyarrhythmie.

Nach dem Start der Prozedur wird in der ersten Berechnungseinheit 203a der zeitliche Abstand zweier aufeinanderfolgender, auf an sich bekannte Weise durch die Ventrikel-Elektrode 1a abgefühlter und im zugeordneten Leseverstärker 201a aufbereiteter R-Wellen-Signale (das RR-Intervall) bestimmt.

In zwei aufeinanderfolgenden Schritten S1 und S2 wird über den Vergleicher 205 zunächst in einem Vergleich mit den im Speicher 204 gespeicherten Intervall-Schwellwerten eine Zuordnung zu einem der Intervall- bzw. Ratenbereiche (1) "normaler" Sinusaktivität (relativ lange Intervall- bzw. niedrige Ratenwerte) oder (2) näher zu klassifizierender Tachyarrhythmiezustände (für einen Tachyarrhythmiezustand mittelgroße Intervall- bzw. Ratenwerte) oder (3) Fibrillations-Bereich (sehr kurze Intervall- bzw. hohe Ratenwerte) vorgenommen. Die beiden Schwellwerte sind patientenspezifisch und vom Arzt zu programmieren.

Wird festgestellt, daß der Intervallwert weder in einem Tachykardiebereich (2) ("Nein" im Schritt S1) noch im Fibrillationsbereich (3)("Nein" im Schritt S2), mithin im Bereich (1), liegt, kann von der Einleitung einer Elektrotherapie abgesehen werden ("Stop" nach S2). Liegt er hingegen im Bereich (3) ("Ja" im Schritt S2), wird in der Regel über den Controller 3, der das entsprechende Klassifizierungssignal $cs_1$ empfangen und verarbeitet hat, der Defibrillator 6 angesteuert und eine Defibrillation bzw. Kardioversion eingeleitet. Ggfs. kann der Arzt auch, nachdem ihm als Klassifizierungsergebnis auf der Anzeige 7 das Vorliegen von Herzkammerflimmern angezeigt wurde, kurzfristig eine Medikamentengabe vornehmen.

Liegt der aktuelle RR-Intervall- bzw. ventrikuläre Ratenwert im Bereich (2) oder (3) ("Ja" im Schritt S1), so wird in einem Schritt S3 zusätzlich geprüft, ob die Tachyarrhythmie plötzlich eingesetzt hat. Hierzu wird der aktuell ermittelte, am Ausgang der ersten Berechnungseinheit 203a verfügbare RR-Intervall-Mittelwert im Komparator 214 einem Vergleich mit dem oder den vorhergehenden Mittelwert(en) unterzogen, der/die aus dem ersten Intervallspeicher 206a abrufbar ist/sind. (Die für die Mittelwertbildung zusätzlich nötigen Funktionselemente sind dem Fachmann vertraut und der besseren Übersichtlichkeit halber in Fig. 1 nicht dargestellt.) Das von dem Komparator 214 abgegebene Klassifizierungssignal $cs_7$ drückt aus, ob eine den im Vergleichswertspeicher 213 gespeicherten zulässigen Differenzbetrag übersteigende Verkürzung des RR-Intervalls vorliegt, die als Beleg für ein plötzliches Einsetzen der Tachyarrhythmie zu werten ist.

Das ein plötzliches Einsetzen bestätigende Klassifizierungssignal $cs_7$ wird (in einem - in Fig. 1 nicht gezeigten Speicherbereich des Controllers 3) gespeichert, bis eine vorbestimmte Anzahl von (z.B. vier) Herzaktivitäten in den Bereich (1) gefallen ist. Es dient als Klassifizierungskriterium in einem weiter unten erwähnten Zustand.

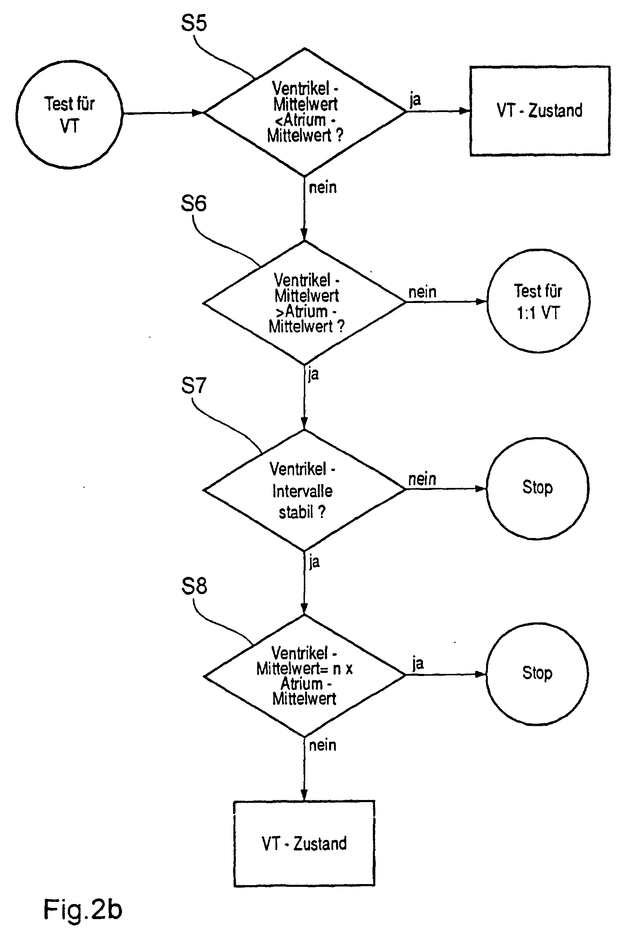

Schritt S4 in Fig. 2a steht dafür, daß nach der Prüfung auf plötzliches Einsetzen nochmals geprüft wird, ob der RR-Intervallwert im o.g. Bereich (3) liegt. Trifft dies zu ("Ja" im Schritt S4), liegt eine ventrikuläre Fibrillation ("VF-Zustand") vor, die so zu therapieren ist wie oben für "Nein" im Schritt S2 erwähnt. Anderenfalls ("Nein" im Schritt S4) werden zur Klassifizierung der Tachyarrhythmie weitere Kriterien geprüft ("Test für VT" - siehe weiter in Fig. 2b). In einem Schritt S5 wird in der Komparatorstufe 207 ein Vergleich der RR-Intervalle (Mittelwerte) mit den am Ausgang der zweiten Berechnungseinheit 203b bereitstehenden PP-Intervallen (Mittelwerten) ausgeführt. Sind die RR-Intervalle kürzer als die PP-Intervalle ("Ja" im Schritt S5), liegt eine ventrikuläre Tachykardie ("VT-Zustand") vor.

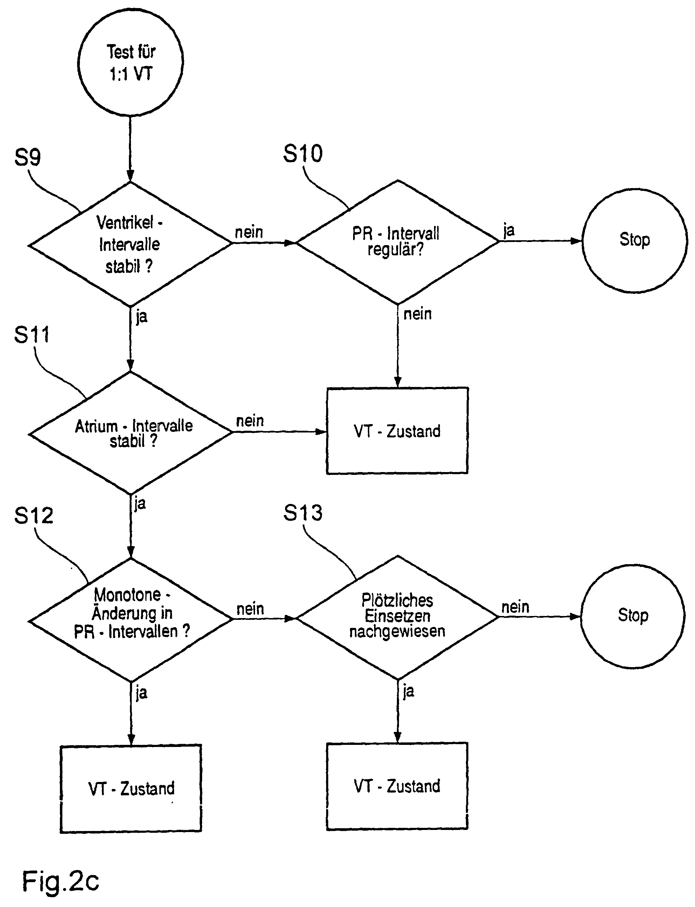

Sind die RR-Intervall-Mittelwerte nicht kürzer als die PP-Intervall-Mittelwerte ("Nein" im Schritt S5), wird im Flußdiagramm zu einem Schritt S6 weitergegangen, in dem geprüft wird, ob sie länger als die PP-Intervall-Mittelwerte sind. In der Praxis kann dieser Schritt in einem Arbeitsgang mit S5 ausgeführt werden. Ist dies nicht der Fall ("Nein" im Schritt S6), wird mit einem "Test für 1:1 VT" fortgefahren - siehe weiter unten und Fig. 2c. Trifft es hingegen zu ("Ja" im Schritt S6), wird zum Schritt S7 weitergegangen.

Im Schritt S7 wird geprüft, ob die RR-Intervalle zeitlich stabil sind. Hierzu wird das Berechnungsergebnis der dritten Berechnungseinheit 208a im Komparator 210a einem Vergleich mit einem (in ms oder %) programmierten, im Kriterienspeicher 209a gespeicherten Stabilitätskriterium unterzogen. In praxi werden bevorzugt die Differenzen zwischen dem aktuellen und jedem der drei vorhergehenden RR-Intervall-Mittelwerte mit dem Stabilitätskriterium verglichen, und Stabilität wird als gegeben angenommen, wenn in allen drei Vergleichen das Kriterium erfüllt ist. Sind die RR- bzw. Ventrikel-Intervalle nicht stabil ("Nein" im Schritt S7), wird der Test mit dem Ergebnis abgebrochen, daß keine VT vorliegt ("Stop").

Sind sie hingegen stabil ("Ja" im Schritt S7), wird zu einem Schritt S8 weitergegangen. Im Schritt S8 wird in der Komparatorstufe 207 geprüft, ob der vom Ausgang der ersten Berechnungseinheit 203a abgegriffene RR-Intervall-Mittelwert ein ganzzahliges Vielfaches des vom Ausgang der zweiten Berechnungseinheit 203b abgegriffenen PP-Intervall-Mittelwertes ist. Dieser Vorgang ist oben unter Bezugnahme auf Fig. 1a beschrieben. Wird festgestellt, daß der RR-Mittelwert ein ganzzahliges Vielfaches des PP-Mittelwertes ist ("Ja" im Schritt S8), so wird der Test abgebrochen ("Stop") - wiederum mit dem Ergebnis, daß keine VT vorliegt. Es handelt sich dann um eine n:1 in den Ventrikel übergeleitete atrielle Tachykardie. Wird hingegen festgestellt, daß er kein ganzzahliges Vielfaches ist ("Nein" im Schritt S8), besteht eine VT.

Schritt S9, der erste Schritt des in Fig. 2a verdeutlichten Tests auf das Vorliegen einer 1:1-ventrikulären Tachykardie, entspricht obigem Schritt S7. Wird im Ergebnis festgestellt, daß die Ventrikel-Intervalle nicht stabil sind ("Nein" im Schritt 5), wird in einem Schritt S1O geprüft, ob die PR-Intervalle regulär sind. Hierzu wird das Berechnungsergebnis der fünften Berechnungseinheit 211 in der Vergleicher- und Berechnungseinheit 212 einem Vergleich mit einem programmierten, im Speicher 209c gespeicherten Regularitätskriterium unterzogen. Hierfür wird in der praktischen Ausführung bevorzugt die Hälfte des Wertes des oben erwähnten Stabilitätskriteriums für die RR-Intervalle angesetzt und der Vergleich für mehrere frühere PR-Intervallwerte ausgeführt. Ergibt sich hierbei Einhaltung der Regularitätsbedingung ("Ja" im Schritt S10), liegt mit Sicherheit keine VT vor und die Prüfung wird abgebrochen. Ist diese hingegen nicht eingehalten, liegt ventrikuläre Tachykardie vor ("VT-Zustand").

Wird hingegen in Schritt S9 festgestellt, daß hinsichtlich der RR-Intervalle Stabilität vorliegt, wird zu einem Schritt S11 weitergegangen, in dem auf die gleiche Weise durch die vierte Berechnungseinheit 208a unter Rückgriff auf die im zweiten Intervallspeicher 206b gespeicherten PP-Intervalle die Stabilität der Atrium- bzw. PP-Intervalle geprüft wird. Erweisen sich diese nicht als stabil, liegt ebenfalls ein VT-Zustand vor. Sind sie hingegen stabil, wird im Ablauf zu einem Schritt S12 weitergegangen.

Im Schritt S12 wird geprüft, ob die zeitliche Änderung der PR-Intervalle monoton ist. Hierzu wird das Berechnungsergebnis der fünften Berechnungseinheit 211 in der Vergleicher- und Berechnungseinheit 212 einem Vergleich mit einem programmierten, im Speicher 209c gespeicherten Monotoniekriterium unterzogen. Dieser Vergleich umfaßt wiederum eine Bewertung der zeitlichen Entwicklung für die vier letzten PR-Intervall-Mittelwerte, wobei das Monotoniekriterium darin besteht, daß in der zeitlichen Reihenfolge aller vier Intervalle jedes folgende Intervall entweder länger oder kürzer als das vorhergehende ist:

$$PR(t1) \sim PR(t2) \leqq PR(t3) \leqq PR(t4) \text{ für } t1<t2<t3<t4$$

oder

$$PR(t1) \sim PR(t2) \sim PR(t3) \sim PR(t4) \text{ für } t1<t2<t3<t4.$$

Liegt in diesem Sinne Monotonie vor ("Ja" im Schritt S12), ist der Zustand als ventrikuläre Tachykardie zu klassifizieren. Liegt keine Monotonie vor ("Nein" im Schritt S12), wird zu einem letzten Schritt S13 weitergegangen. Im Schritt S13 wird auf die gespeicherte Aussage zum Vorliegen/Nichtvorliegen eines plötzlichen Einsetzens der Tachyarrhythmie (siehe oben zu S3) zugegriffen. Lag kein solches vor, liegt - unter den übrigen Voraussetzungen der bis hierher durchlaufenen Schritte - auch keine VT vor. War hingegen plötzliches Einsetzen zu verzeichnen, handelt es sich bei der Arrhythmie - bei Erfüllung der übrigen Kriterien - um eine VT.

Die definitive Aussage über das Vorliegen einer ventrikulären Tachykardie wird getroffen, wenn ein mehrmaliges Durchlaufen der obigen Prozedur (zu verschiedenen Zeitpunkten innerhalb eines Prüfungszeitraumes, mit den jeweils gültigen Intervallwerten) die Erfüllung der Kriterien für den "VT-Zustand" ergeben hat, ohne daß zwischenzeitlich ein "normaler" Ventrikelzustand ("Stop" in Fig. 2a) festgestellt wurde. Wird ein "normaler" Zustand festgestellt, wird der Zählwert von "VT-Zustand" auf Null gesetzt und die Prozedur von vorn begonnen.

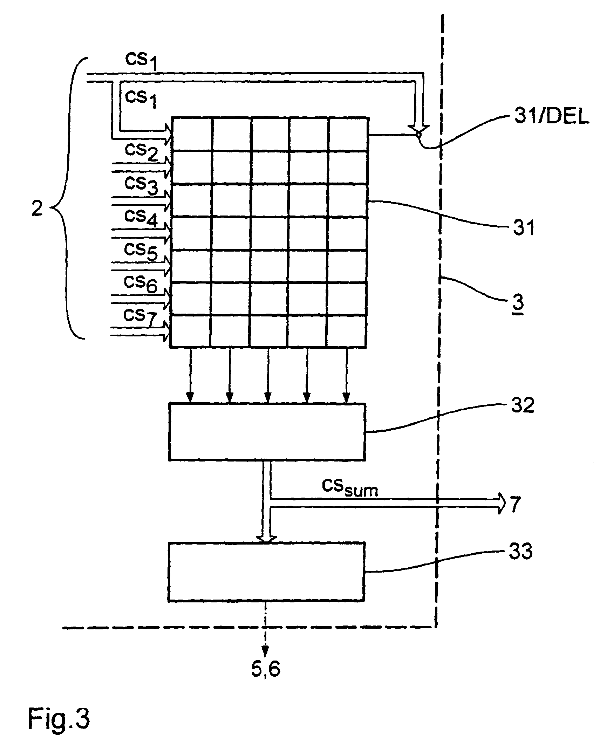

Die Ablaufsteuerung 3 aus Fig. 1 ist daher - wie Fig. 3 zeigt - bevorzugt so ausgebildet, daß ein Teilergebnisspeicher 31 mit 7 Zeilen und x Spalten, in dem eine vorbestimmte Anzahl x aufeinanderfolgender Testdurchläufe gespeichert werden kann, vorgesehen ist und die zugehörigen x letzten Sätze von Klassifizierungssignalen $cs_1$ bis $cs_7$ gemäß Fig. 1a <und/oder - was in Fig. 3 nicht gezeigt ist - das daraus abgeleitete jeweilige Bewertungsergebnis "VT-Zustand" gemäß Fig. 2a bis 2c) gespeichert wird. Mit dem Ausgang des nach dem FIFO-Prinzip organisierten Speichers 31 ist eine logische Verknüpfungseinheit (ein mehrstufiges UND-Gatter) 32 verbunden, die nach jedem neuen Durchlauf die Ergebnisse der letzten x Durchläufe zu einem Gesamtaussage-Signal $CS_{sum}$ verknüpft, das einer Schaltsteuereinheit 33 für die angeschlossenen Therapiegeräte 5, 6 zugeführt wird. Das Signal $CS_1$ gelangt zusätzlich auf einen Löscheingang des Speichers 31, der dadurch gelöscht wird, wenn $CS_1$ im aktuellen Durchlauf einen Wert hat, der den oben erwähnten Bereich (1) repräsentiert.

Zumindest das Gesamtergebnis kann auch auf der Anzeigeeinheit 7 dargestellt werden; es können aber auch die Klassifizierungssignale und/oder Bewertungen für jeden Testdurchlauf einzeln angezeigt werden, so daß der Arzt anhand der Anzeige eine subjektive Gesamtwertung vornehmen und im Zweifelsfall die Prüfung ausdehnen oder wiederholen kann.

Die definitive Aussage über das Vorliegen ventrikulärer Fibrillation wird vorzugsweise getroffen, wenn bei y Messungen innerhalb einer Gesamtzahl von z gemessenen ventrikulären Intervallen - wobei y und z programmierbar sind - der Zustand ventrikulärer Fibrillation ("VF-Zustand") festgestellt wurde.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch in anders gearteter Ausführung Gebrauch macht.

So kann die Abfolge der oben skizzierten Schritte modifiziert sein, und in Abhängigkeit von spezifischen Patientenbedingungen können auch bestimmte Schritte im Ablauf durch entsprechende Programmierung übergangen werden. Die angegebenen Funktionselemente können in einer anderen Konfiguration auch durch ähnlich wirkende ersetzt sein.

**Patentansprüche**

**1.** Vorrichtung (2, 3) zum Nachweis ventrikulärer Tachykardie, mit

- einem Atrium-Fühler (ib, 201b) zum Abfühlen elektrischer Aktivität im Vorhof (A) und einem Ventrikel-Fühler (1a, 201a) zum Abfühlen elektrischer Aktivität in der Herzkammer (V) eines Herzens (H),

- einem Zeitgeber (202) zur Erfassung des Zeitpunktes des Auftretens der jeweiligen elektrischen Aktivität,

- einer eingangsseitig mit dem Ventrikel-Fühler (1a, 201a) bzw. dem Atrium-Fühler (ib, 201b) und dem Zeitgeber/den Zeitgebern (202) verbundenen ersten und zweiten Berechnungseinheit (203a, 203b) zur Berechnung der RR- bzw. PP-Intervalle zwischen jeweils aufeinanderfolgenden elektrischen Aktivitäten im Vorhof (A) bzw. in der Herzkammer (V) und wahlweise zur Bildung von Intervall-Mittelwerten über einen vorbestimmten Zeitraum oder eine vorbestimmte Anzahl berechneter RR- bzw. PP-Intervalle,

- einem Schwellwertspeicher (204) zur Speicherung mindestens eines Schwellwertes für RR-Intervalle bzw. Intervall-Mittelwerte,

- einer mit den Ausgängen der ersten Berechnungseinheit (203a) und des Schwellwertspeichers (204) verbundenen ersten Vergleichereinheit (205) zum Vergleich der RR-Intervalle oder RR-Intervall-Mittelwerte mit den Schwellwerten und zur Ausgabe eines das Vergleichsergebnis kennzeichnenden ersten Klassifizierungssignals ($cs_1$),

- einem mit dem Ausgang der ersten bzw. zweiten Berechnungseinheit verbundenen ersten bzw. zweiten Intervallspeicher (206a, 206b) zur Speicherung jeweils einer vorbestimmten Anzahl berechneter RR- bzw. PP-Intervalle oder Intervall-Mittelwerte,

- einer mit dem Ausgang der ersten und zweiten Berechnungseinheit oder der ersten und zweiten Speichereinheit verbundenen zweiten Vergleichereinheit (207) zum Vergleich der PP-Intervalle mit den RR-Intervallen und zur Ausgabe eines das Vergleichsergebnis kennzeichnenden zweiten Klassifizierungssignals ($cs_2$),

- einer mit dem Ausgang der ersten und zweiten Berechnungseinheit und/oder der ersten bzw. zweiten Speichereinheit verbundenen dritten und vierten Berechnungseinheit (208a, 208b) zur Berechnung der zeitlichen Änderungen zwischen aufeinanderfolgenden PP- und RR-Intervallen oder Intervall-Mittelwerten,

- einem Kriterienspeicher (209a, 209b) zur Speicherung eines vorgegebenen Stabilitätskriteriums für die zeitliche Änderung zwischen aufeinanderfolgenden PP- und RR-Intervallen oder Intervall-Mittelwerten und

- einer eingangsseitig mit dem Ausgang der dritten bzw. vierten Berechnungseinheit und dem Kriterienspeicher verbundenen dritten bzw. vierten Vergleichereinheit (210a, 210b) zum Vergleich der berechneten zeitlichen Änderung der PP- bzw. RR-Intervalle mit dem gespeicherten zugehörigen Stabilitätskriterium und zur Ausgabe eines das Vergleichsergebnis kennzeichnenden dritten und vierten Klassifizierungssignals ($cs_3$, $cs_4$).

2. Vorrichtung nach Anspruch 1, **dadurch gekennzeichnet, daß** die zweite Vergleichereinheit (207) einen Dividierer (207.1) mit einem Quotienten- und einem Toleranzbereichspeicher (207.3, 207.4) und nachgeschalteter Vergleicherstufe (207.2) aufweist, wobei die Eingänge des Dividierers mit den Ausgängen der ersten und zweiten Berechnungseinheit (203a, 203b) oder der ersten und zweiten Speichereinheit (206a, 206b), der Ausgang des Dividierers (207.1) mit einem Eingang der Vergleicherstufe (207.2) und der Ausgang des Quotienten- und Toleranzbereichspeichers (207.2, 207.4) mit weiteren Eingängen der Vergleicherstufe verbunden sind derart, daß der Dividierer eine Quotientenbildung aus gespeicherten RR- und PP-Intervallen oder Intervall-Mittelwerten ausführt und der berechnete Quotient in der Vergleicherstufe daraufhin geprüft wird, ob er innerhalb eines vorgegebenen Toleranzbereiches eine ganze Zahl darstellt.

3. Vorrichtung nach Anspruch 1 oder 2, **gekennzeichnet durch**

- eine eingangsseitig mit dem Atrium-Fühler (1b, 201b) und dem Ventrikel-Fühler (1a, 201a) und dem Zeitgeber bzw. den Zeitgebern (202) verbundenen fünften Berechnungseinheit (203c) zur Berechnung der PR-Intervalle zwischen jeweils einer elektrischen Aktivität im Vorhof und der zeitlich nächstfolgenden Aktivität in der Herzkammer und wahlweise zur Bildung eines Intervall-Mittelwertes über einen vorbestimmten Zeitraum oder eine vorbestimmte Anzahl berechneter Zeitintervalle,

- einen mit dem Ausgang der fünften Berechnungseinheit (203c) verbundenen dritten Intervallspeicher (206c) zur Speicherung jeweils einer vorbestimmten Anzahl berechneter PR-Intervalle oder Intervall-Mittelwerte,

- eine mit dem Ausgang der fünften Berechnungseinheit und/oder dem dritten Intervallspeicher verbundene sechste Berechnungseinheit (211) zur Berechnung der zeitlichen Änderungen zwischen aufeinanderfolgenden PR-Intervallen oder Intervall-Mittelwerten,

- einem dritten Kriterienspeicher (209c) zur Speicherung eines vorgegebenen Regularitäts- und wahlweise Monotoniekriteriums für die zeitliche Änderung der PR-Intervalle oder Intervall-Mittelwerte und

- einer eingangsseitig mit dem Ausgang der sechsten Berechnungseinheit (211) und dem dritten Kriterienspeicher (209c) verbundenen fünften Vergleichereinheit (212) zum Vergleich der berechneten zeitlichen Änderung der PR-Intervalle mit dem gespeicherten Regularitäts- bzw. Monotoniekriterium und zur Ausgabe eines das Vergleichsergebnis kennzeichnenden fünften bzw. sechsten Klassifizierungssignals ($cs_5$, $cs_6$).

4. Vorrichtung nach einem der vorhergehenden Ansprüche, **dadurch gekennzeichnet, daß** ein Vergleichswertspeicher (213) zur Speicherung eines RR-Vergleichsintervalles oder eines RR-Abweichungsbetrages und eine eingangsseitig mit den Ausgängen der ersten Berechnungseinheit (203a) und des ersten Intervallspeichers (206a) sowie des Vergleichswertspeichers verbundene siebente Vergleichereinheit (214) zum Vergleich des aktuellen RR-Intervalls oder Intervall-Mittelwertes mit dem gespeicherten RR-Vergleichsintervall oder einem vorhergehenden RR-Intervall unter Berücksichtigung des RR-Abweichungsbetrages und zur Ausgabe eines das Vergleichsergebnis kennzeichnenden siebenten Klassifizierungssignals ($cs_7$) zugeordnet ist.

5. Vorrichtung nach Anspruch 4, **dadurch gekennzeichnet, daß** dem Vergleichswertspeicher (213) eingangsseitig eine mit dem Ausgang des ersten Intervallspeichers (206a) verbundene Vergleichswert-Berechnungseinheit (215) zugeordnet ist, die laufend anhand des aktuellen RR-Intervalles das RR-Vergleichsintervall oder den RR-Abweichungsbetrag berechnet.

6. Vorrichtung nach einem der vorhergehenden Ansprüche, **gekennzeichnet durch** eine Ablaufsteuereinrinchtung (3) mit Programmspeicher (4) zur Steuerung des Betriebs der Vorrichtung gemäß einem gespeicherten Flußdiagramm unter sequentieller Abfrage und Verarbeitung der Klassifizierungssignale zu Steuersignalen für einzelne Komponenten der Vorrichtung und/oder zur selbsttätigen Aktivierung eines dieser zugeordneten Herztherapiegerätes (5, 6).

7. Vorrichtung nach einem der vorhergehenden Ansprüche, **dadurch gekennzeichnet, daß** die erste und/oder zweite und/oder fünfte Berechnungseinheit (203a, 203b, 203c) einen Intervallzahlspeicher (203a.2) zur Speicherung einer vorgegebenen Anzahl von für eine Zeitmittelung zu erfassenden Intervallen, jeweils eine mit dem Ausgang des Speichers verbundene Additions- bzw. Integrationsstufe (203a.1) zum Bestimmen der Gesamtdauer der gespeicherten Anzahl aufeinanderfolgender Intervalle und jeweils eine mit dem Ausgang der Additions- bzw. Integrationsstufe sowie des Intervallzahlspeichers verbundene Divisionsstufe (203a.3) zur Bildung des jeweiligen Intervall-Mittelwertes aufweist.

8. Vorrichtung nach einem der vorhergehenden Ansprüche, **dadurch gekennzeichnet, daß** der erste bis dritte Intervallspeicher (206a bis 206c) jeweils mindestens drei getrennte Speicherbereiche aufweist.

9. Vorrichtung nach einem der vorhergehenden Ansprüche, **dadurch gekennzeichnet, daß** die dritte und/oder vierte und/oder sechste Berechnungseinheit (208a, 208b, 211) zur Ausführung einer Trendberechnung der zeitlichen Entwicklung zwischen mehr als zwei der in der jeweils eingangsseitig zugeordneten Berechnungseinheit berechneten bzw. Speichereinheit gespeicherten Zeitintervalle ausgebildet ist.

10. Vorrichtung nach einem der vorhergehenden Ansprüche, **dadurch gekennzeichnet, daß** die Ablaufsteuereinrichtung (3) einen Teilergebnisspeicher (31) mit mehreren Spalten und einem Löscheingang (31/DEL) aufweist, wobei

die in einem von einer vorbestimmten Anzahl aufeinanderfolgender Prüfdurchgänge gewonnenen Klassifizierungssignale ($cs_1$ bis $cs_7$) und/oder Zustandsaussagen jeweils einer Spalte zugeführt werden und der gesamte Speicherinhalt bei Anliegen eines einen bestimmten Wert aufweisenden ersten Klassifizierungssignals ($cs_1$) selbsttätig gelöscht wird und

die Ausgänge der einzelnen Spalten getrennt mit Eingängen einer logischen Verarbeitungseinheit (32) verbunden sind, in der die Speicherinhalte jeweils nach einem Prüfdurchgang spaltenweise verküpft werden und daraus ein Gesamtbewertungssignal ($CS_{sum}$) hinsichtlich des Vorliegens einer ventrikulären Tachykardie (VT-Zustand) gewonnen wird.

## Claims

1. Apparatus (2, 3) for detecting ventricular tachycardia, comprising

- an atrium sensor (1b, 201b) for sensing electrical activity in the atrium (A) and a ventricle sensor (1a, 201a) for sensing electrical activity in the ventricle (V) of a heart (H),
- a timer (202) for detecting the moment of the occurrence of the respective electrical activity,
- a first and a second calculation unit (203a, 203b) connected at the input side to the ventricle sensor (1a, 201a) and the atrium sensor (1b, 201b) respectively and the timer/timers (202) for calculation of the RR-and PP-intervals respectively between respectively successive electrical activities in the atrium (A) and in the ventricle (V) and selectively for forming interval averages over a predetermined period of time or over a predetermined number of calculated RR- and PP-intervals respectively,

- a threshold value store (204) for the storage of at least one threshold value in respect of RR-intervals and interval averages respectively,
- a comparator unit (205) connected to the outputs of the first calculation unit (203a) and the threshold value store (204) for comparing the RR-intervals or RR-interval averages respectively to the threshold values and for outputting a first classification signal ($CS_1$) characterising the comparison result,
- a first and a second interval store (206a, 206b) connected to the output of the first and second calculation unit respectively for the storage of a respective predetermined number of calculated RR- and PP-intervals or interval averages,
- a second comparator unit (207) connected to the output of the first and second calculation unit or the first and second storage unit for comparison of the PP-intervals to the RR-intervals and for output of a second classification signal ($cs_2$) characterising the comparison result,
- a third and a fourth calculation unit (208a, 208b) connected to the output of the first and second calculation unit and/or the first and second storage unit respectively for calculation of the time changes between successive PP- and RR-intervals or interval averages,
- a criterion store (209a, 209b) for storage of a predetermined stability criterion for the time change between successive PP- and RR-intervals or interval averages, and
- a third and a fourth comparator unit (201a, 201b) connected at the input side to the output of the third and fourth calculation unit and the criterion store for comparison of the calculated time change of the PP- and RR-intervals respectively with the stored associated stability criterion and for the output of a third and fourth classification signal ($cs_3$, $cs_4$) characterising the comparison result.

2. Apparatus according to claim 1 **characterised in that** the second comparator unit (207) has a divider (207.1) with a quotient and a tolerance range store (207.3, 207.4) and a downstream-connected comparator stage (207.2), wherein the inputs of the divider are connected to the outputs of the first and second calculation units (203a, 203b) or the first and second store units (206a, 206b), the output of the divider (207.1) is connected to an input of the comparator stage (207.2) and the output of the quotient and tolerance range store (207.2, 207.4) is connected to further inputs of the comparator stage, in such a way that the divider executes a quotient formation operation from stored RR- and PP-intervals or interval averages and the calculation quotient is checked in the comparator stage to ascertain whether it represents a whole number within a predetermined tolerance range.

3. Apparatus according to claim 1 or claim 2 **characterised by**

- a fifth calculation unit (203c) connected at the input side to the atrium sensor (1b, 201b) and the ventricle sensor (1a, 201a) and the timer or timers (202) for calculation of the PR-intervals between a respective electrical activity in the atrium and the activity which next follows in respect of time in the ventricle and selectively for forming an interval average over a predetermined period of time or over a predetermined number of calculated time intervals,
- a third interval store (206c) connected to the output of the fifth calculation unit (203c) for storage of a respective predetermined number of calculated PR-intervals or interval averages,
- a sixth calculation unit (211) connected to the output of the fifth calculation unit and/or the third interval store for calculation of the time changes between successive PR-intervals or interval averages,
- a third criterion store (209c) for storage of a predetermined regularity and optionally monotony criterion for the time change of the PR-intervals or interval averages, and
- a fifth comparator unit (212) connected at the input side to the output of the sixth calculation unit (211) and the third criterion store (209c) for comparison of the calculated time change of the PR-intervals with the stored regularity or monotony criterion respectively and for the output of a fifth and a sixth classification signal ($cs_5$, $cs_6$) characterising the comparison result.

4. Apparatus according to one of the preceding claims **characterised in that** there is associated a comparison value store (213) for storing an RR-comparison interval or an RR-deviation amount and a seventh comparator unit (214) connected at the input side to the outputs of the first calculation unit (203a) and the first interval store (206a) as well as the comparison value store for calculation of the current RR-interval or interval average with the stored RR-comparison interval or a preceding RR-interval having regard to the RR-deviation amount and for the output of a seventh classification signal ($cs_7$) characterising the comparison result.

5. Apparatus according to claim 4 **characterised in that** associated with the comparative value store (213) at the input side is a comparative value calculation unit (215) which is connected to the output of the first interval store (206a) and which continuously calculates the RR-comparison interval or the RR-deviation amount on the basis of the current RR-interval.

6. Apparatus according to one of the preceding claims **characterised by** an operational control device (3) with program store (4) for controlling operation of the apparatus in accordance with a stored flow chart with sequential interrogation and processing of the classification signals to afford control signals for individual components of the apparatus and/or for independent activation of a cardiac therapy unit (5, 6) associated therewith.

7. Apparatus according to one of the preceding claims **characterised in that** the first and/or second and/or fifth calculation unit (203a, 203b, 203c) has an interval number store (203a.2) for storage of a predetermined number of intervals to be detected for time averaging, a respective addition or integration stage (203a.1) connected to the output of the store for determining the total duration of the stored number of successive intervals and a respective division stage (203a.3) connected to the output of the addition or integration stage and the interval number store for forming the respective interval average.

8. Apparatus according to one of the preceding claims **characterised in that** the first to third interval stores (206a to 206c) each have at least three separate store regions.

9. Apparatus according to one of the preceding claims **characterised in that** the third and/or fourth and/or sixth calculation unit (208a, 208b, 211) is adapted to perform a trend calculation in respect of the development in respect of time between more than two of the time intervals calculated in the calculation unit respectively associated at the input side or stored in the store unit.

10. Apparatus according to one of the preceding claims **characterised in that** the operational control device (31) has a partial result store (31) with a plurality of columns and a delete input (31/DEL), wherein the classification signals ($cs_1$ to $cs_7$) and/or condition information obtained in one of a predetermined number of successive testing operations are fed to a respective column and the entire store content is automatically deleted upon the application of a first classification signal ($cs_1$) of a given value, and the outputs of the individual columns are connected separately to inputs of a logic processing unit (32) in which the store contents are respectively linked column-wise after a testing operation and an overall evaluation signal ($CS_{sum}$) is obtained therefrom in respect of the existence of a ventricular tachycardia (VT-condition).

## Revendications

1. Dispositif (2, 3) pour détecter une tachycardie ventriculaire, comprenant

   - un capteur d'oreillette (1b, 201b) pour détecter une activité électrique dans l'oreillette (A) et un capteur de ventricule (1a, 201a) pour détecter une activité électrique dans la cavité cardiaque (V) d'un coeur (H),
   - une minuterie (202) pour détecter l'instant d'apparition de l'activité électrique respective,
   - des première et seconde unités de calcul (203a, 203b) reliées côté entrée au capteur d'entrée (1a, 201a) ou au capteur d'oreille (1b, 201b) et la ou les minuteries (202), pour le calcul des intervalles RR et PP entre des activités électriques respectivement successives dans l'oreillette (A) et dans la cavité cardiaque (V) et au choix pour la formation de valeurs moyennes d'intervalles pendant un intervalle de temps prédéterminé ou pendant un nombre prédéterminé d'intervalles RR ou PP calculé,
   - une mémoire de valeur de seuil (204) pour mémoriser au moins une valeur de seuil pour des valeurs moyennes des intervalles RR et des intervalles PP,
   - une première unité de comparaison (205) relié aux sorties de la première unité de calcul (203a) et de la mémoire de valeur de seuil (205) pour comparer les valeurs moyennes des intervalles RR ou des intervalles PP aux valeurs de seuil et pour délivrer un premier signal de classification ($cs_1$) caractérisant le résultat de la comparaison,
   - des première et seconde mémoires d'intervalles (206a, 206b) reliés à la sortie des première et seconde unités de calcul pour mémoriser respectivement un nombre prédéterminé d'intervalles RR ou d'intervalles PP calculés ou de valeurs moyennes d'intervalles,
   - une seconde unité de comparaison (207) reliée à la sortie des première et seconde unités de calcul ou des première et seconde unités de mémoire pour comparer les intervalles PP aux intervalles RR et pour délivrer un deuxième signal de classification ($cs_2$) caractérisant le résultat de la comparaison,
   - des troisième et quatrième unités de calcul (208a, 208b) reliées à la sortie des première et seconde unités de calcul et/ou de la première ou de la seconde unité de mémoire pour calculer les variations dans le temps entre des intervalles PP et des intervalles RR successifs ou des valeurs moyennes successives d'intervalles,
   - une mémoire de critère (209a, 209b) pour mémoriser un critère de stabilité prédéterminé pour la variation dans le temps entre des intervalles PP et des intervalles RR successifs ou des valeurs moyennes successives d'intervalles, et

- des troisième et quatrième unités de comparaison (210a, 210b) reliées côté entrée à la sortie des troisième et quatrième unités de calcul et à la mémoire de critère pour comparer la variation dans le temps calculée, des intervalles PP ou des intervalles RR au critère de stabilité mémorisé associé et pour délivrer les troisième et quatrième signaux de classification ($cs_3$, $cs_4$) caractérisant le résultat de la comparaison.

2. Dispositif selon la revendication 1, **caractérisé en ce que** la seconde unité de comparaison (207) comporte un diviseur (207.1) comportant une mémoire de quotients et une mémoire de plages de tolérance (207.3, 207.4) et un étage comparateur (207.2) branché en aval, les entrées du diviseur étant liées aux sorties des première et seconde unités de calcul (203a, 203b) ou des première et seconde unités de mémoire (206a, 206b), la sortie du diviseur (207.1) étant reliée à une entrée de l'étage comparateur (207.2) et la sortie des mémoires de quotients et de plages de tolérance (207.2, 207.4) étant reliées à d'autres entrées de l'étage comparateur, de telle sorte que le diviseur forme un quotient à partir des intervalles d'erreur et des intervalles PP mémorisés ou des valeurs moyennes mémorisées d'intervalles et que le quotient calculé est contrôlé dans l'étage comparateur pour déterminer s'il représente un nombre entier, dans les limites d'une plage prédéterminée de tolérance.

3. Dispositif selon la revendication 1 ou 2, **caractérisé par**

- une cinquième unité de calcul (203c) reliée côté entrée au capteur d'oreillette (1b, 201b) et au capteur de ventricule (1a, 201a) et à la ou aux minuteries (202) pour calculer les intervalles PP entre respectivement une activité électrique dans l'oreillette et l'activité immédiatement suivante dans le temps dans la cavité cardiaque et au choix pour former une valeur moyenne d'intervalles sur un intervalle de temps prédéterminé ou sur un nombre prédéterminé d'intervalles de temps calculés,
- une troisième mémoire d'intervalles (206c) reliée à la sortie de la cinquième minuterie de calcul (203c) et servant à mémoriser respectivement un nombre prédéterminé d'intervalles PR calculés ou de valeurs moyennes calculées d'intervalles,
- une sixième unité de calcul (211) reliée à la sortie de la cinquième unité de calcul et/ou aux mémoires d'intervalles pour calculer les variations dans le temps entre des intervalles PR successifs ou des valeurs moyennes successives d'intervalles,

- un troisième mémoire de critère (209c) pour mémoriser un critère prédéterminé de régularité ou au choix de monotonie pour la variation dans le temps des intervalles PR ou des valeurs moyenne d'intervalles, et
- une cinquième unité de comparaison (212) reliée côté entrée à la sortie de la sixième unité de calcul (211) et à la troisième mémoire de critère (209c) pour comparer la variation dans le temps calculée des intervalles PR au critère mémorisé de régularité ou de monotonie et pour délivrer un cinquième ou un sixième signal de classification ($cs_5$, $cs_6$) caractérisant le résultat de la comparaison.

4. Dispositif selon l'une des revendications précédentes, caractérisé en qu'une mémoire de comparaison (213) servant à mémoriser un intervalle de comparaison RR ou une valeur d'écart RR et une septième unité de comparaison (214) relié côté entrée aux sorties de la première unité de calcul (203a) et la première mémoire d'intervalles (206a) ainsi que de la mémoire de valeur de comparaison est associé à la comparaison de l'intervalle RR actuel ou la valeur moyenne actuelle d'intervalles à l'intervalle de comparaison RR mémorisé ou à un intervalle RR prédéterminé en tenant compte de la valeur de l'écart RR et servant à délivrer un septième signal de classification ($cs_7$) caractérisant le résultat de la comparaison.

5. Dispositif selon la revendication 4, **caractérisé en ce qu'**à la mémoire de valeur de comparaison (213) est associée, côté entrée, une unité de calcul de valeur de comparaison (215), qui est reliée à la sortie de la première mémoire d'intervalles (206a) et qui calcule en permanence l'intervalle de comparaison (RR) ou la valeur d'écart RR sur la base de l'intervalle RR actuel.

6. Dispositif selon l'une des revendications précédentes, **caractérisé par** un dispositif (3) de commande d'exécution comportant une mémoire programme (4) pour commander le fonctionnement du dispositif conformément à un organigramme mémorisé, moyennant une interrogation et un traitement séquentiels des signaux de classification pour former des signaux de commande pour des composants individuels du dispositif et/ou l'activation automatique d'un appareil de thérapie cardiaque (5, 6) associé à ce dispositif.

7. Dispositif selon l'une des revendications précédentes, **caractérisé en ce que** la première et/ou la seconde et/ou la cinquième unités de calcul (203a, 203b, 203c) d'une mémoire du nombre d'intervalles (203.2) pour mémoriser un nombre prédéterminé d'intervalles devant être détectés pour la formation de la moyenne dans le temps, respectivement à un étage d'addition ou d'intégration (203a.1) relié à la sortie de la mémoire et servant à déterminer la durée totale du nombre mémoriser d'intervalle successif et respectivement un étage de division (203.3) relié à la sortie de l'étage d'addition ou d'intégration de la mémoire du nombre d'intervalles, pour la formation de la valeur moyenne respective d'intervalles.

8. Dispositif selon l'une des revendications précédentes, **caractérisé en ce que** les première à troisième mémoires d'intervalles (206a à 206c) possèdent respectivement au moins trois zones de mémoire séparées.

9. Dispositif selon l'une des revendications précédentes, **caractérisé en ce que** la troisième et/ou la quatrième et/ou la sixième unités de calcul (208a, 208b, 211) est conçue pour l'exécution d'un calcul de tendance du développement temporel entre plus de deux intervalles de temps calculés dans l'unité de calcul associé respectivement côté entrée ou un calcul mémorisés dans l'unité de mémoire associé respectivement côté entrée.

10. Dispositif selon l'une des revendications précédentes, **caractérisé en ce que** le dispositif de commande d'exécution (3) comporte une mémoire (31) de résultats partiels comportant plusieurs colonnes et une entrée d'effacement (31/DEL) et

les signaux de classification ($cs_1$ à $cs_7$) et/ou des indications d'état, qui sont obtenus à partir d'un nombre prédéterminé de procédures de contrôle successives, sont envoyés respectivement à une colonne et l'unité de mémoire complète est effacée automatiquement lors de l'application d'un premier signal de classification ($cs_1$) possédant une valeur déterminée, et

les sorties des différentes colonnes sont reliées séparément à des entrées d'une unité de traitement logique (32) dans laquelle les contenus de mémoire sont combinés respectivement colonne par colonne respectivement après l'exécution d'un contrôle et, à partir de là, le signal d'évaluation global ($cs_{somme}$) concernant la présence d'une tachycardie ventriculaire (état VT) est obtenu.

Fig.1

201b   203b

206b  208b
207   209b        210b

A  1b
203c        206c  211        212
209c

201a  203a        208a
206a
209a        210a
214

202              215  213

204   205

2

cs₃

cs₂

cs₅
cs₆

cs₄

cs₇

cs₁

7

3

5

6

4

8

EP 0 879 621 B1

14

H  V  1a

EP 0 879 621 B1

Fig.1a

201a          203a.1                    203a.3

Σ

202          203a.2                     203a

Fig.1b

**Start**

S1

Ventrikel - Mittelwert in einem Tachykardie- bereich?

nein →

S2

Ventrikel - Intervall in Fibrillations- bereich

nein → **Stop**

ja ↓

S3

Test für plötzliches Einsetzen

S4

Ventrikel - Intervall in Fibrillations- bereich

ja → VF - Zustand

nein ↓

Test für VT

**Fig.2a**

S5

Test für VT

Ventrikel - Mittelwert <Atrium - Mittelwert ?

ja → VT - Zustand

nein

S6

Ventrikel - Mittelwert >Atrium - Mittelwert ?

nein → Test für 1:1 VT

ja

S7

Ventrikel - Intervalle stabil ?

nein → Stop

ja

S8

Ventrikel - Mittelwert= n x Atrium - Mittelwert

ja → Stop

nein

VT - Zustand

Fig.2b

Fig.2c

Fig.3